(19) Europäisches Patentamt / European Patent Office / Office européen des brevets

(11) **EP 1 780 539 A1**

(12) **EUROPEAN PATENT APPLICATION**

(43) Date of publication:
**02.05.2007 Bulletin 2007/18**

(51) Int Cl.:
*G01N 29/07* (2006.01)   *G01N 29/38* (2006.01)
*G01N 29/44* (2006.01)   *G01N 33/38* (2006.01)

(21) Application number: **05256645.2**

(22) Date of filing: **26.10.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Fujimitsu Engineering Co., Ltd.**
**Nishinomiya City**
**Hyogo 662-0977 (JP)**

(72) Inventor: **Hara, Tooru**
**Kobe City**
**Hyogo, 657-0804 (JP)**

(74) Representative: **Billington, Lawrence Emlyn**
**Haseltine Lake**
**Lincoln House**
**300 High Holborn**
**London WC1V 7JH (GB)**

(54) **Method and apparatus for non-destructive ultrasonic testing of concrete structures**

(57)     An ultrasonic pulse is applied from a transmission probe for longitudinal waves kept in contact with the external surface of a concrete structure into the interior of the structure, and a reflected wave returning after being reflected by the boundary face of the concrete structure is detected with a reception probe for longitudinal waves kept in contact with the external surface of a concrete structure. As the first peak recognized in the waveform of the reflected wave represents the reaching time T1 of the bottom face echo which has undergone no mode conversion, and the subsequently obtained second peak, in about 1. 3 to 1. 7 times when first peak appeared, represents the reaching time T2 of the delayed echo which has undergone a mode conversion, the sonic velocity ratio R of longitudinal wave to transverse wave is calculated from these reaching times T1 and T2. Then, object physical quantities corresponding to the sonic velocity ratio R are figured out from correlations known in advance regarding the concrete structure between the sonic velocity ratio R and object physical quantities.

FIG. 1

Printed by Jouve, 75001 PARIS (FR)

**Description**

BACKGROUND OF THE INVENTION

Field of the Invention

The present invention relates to a non-destructive testing method and apparatus for measuring object physical quantities of a concrete structure, such as compressive strength thereof and progress of alkali aggregate reaction therein, in a non-destructive way.

Description of the Related Art

Concrete structures are subject to early deterioration due to such causes as insufficient compressive strength ensuing from faults in work execution, salt damage and alkali aggregate reaction, and such structures are measured by various methods to detect any such defect theymay involve. Among these methods for diagnosing concrete structures, ultrasonic non-destructive testing is given attention for its quickness and low cost.
One example of method to measure the progress of alkali aggregate reaction in concrete structures by ultrasonic probing is disclosed in Japanese Patent Application Laid-open No. 2003-302382. By this method, sonic velocities measured in a plurality of positions in a concrete structure by an ultrasonic reflection method (the velocities of the ultrasonic wave propagating and returning reflected by the different positions in the structure) are averaged, transmission sonic velocities measured by an ultrasonic transmission method in the same positions are also averaged, and the extents of progress of alkali aggregate reactions in these measuring positions are estimated on the basis of the correlation between the ratio of (or the difference between) the two averages and the progress of alkali aggregate reaction.
Since the measuring method referred to above requires measurement of transmission sonic velocities by ultrasonic transmission, if the concrete structure to be measured is a sheathing revetment or a weir whose rear face is buried in soil or sand, core boring will be needed, and therefore this method has room for improvement in terms of the quickness of measurement and cost. Thus, there is not yet any completely non-destructive technique requiring no core boring available for practical use that permits measurement of object physical quantities of an existing concrete structure to which ultrasonic transmission cannot be applied.

SUMMARY OF THE INVENTION

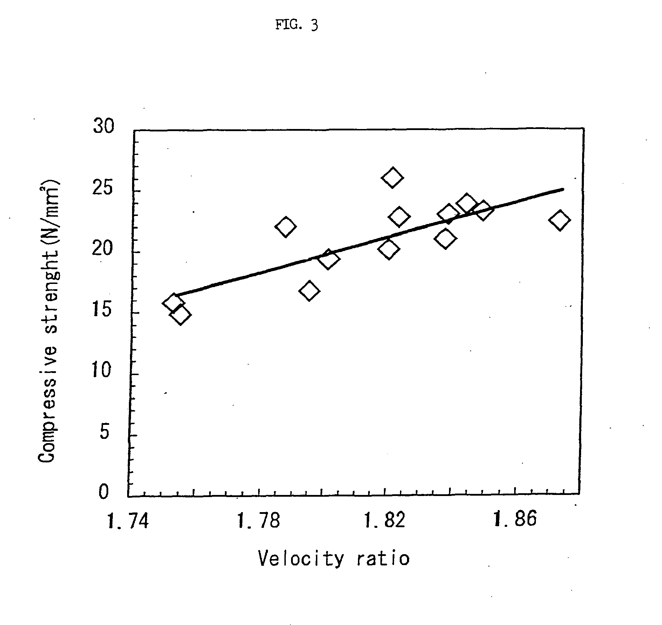

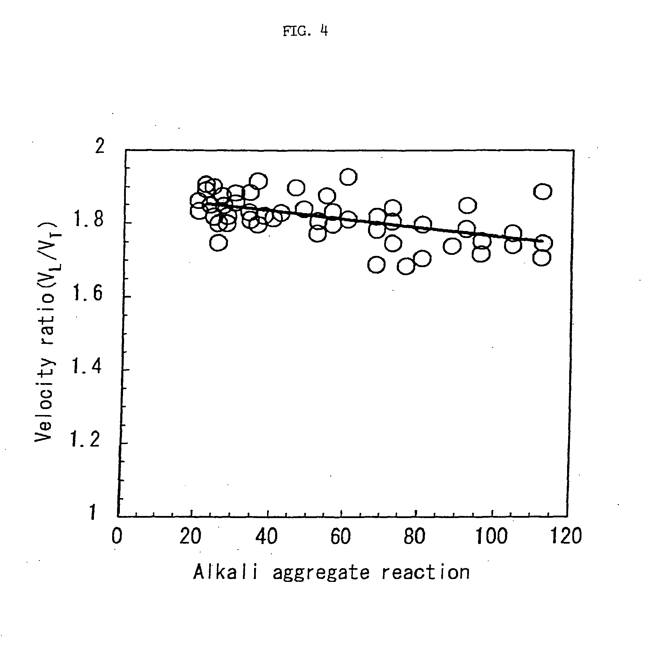

Incidentally, the present inventor conducted laboratory measurement by the transmission method of the sonic velocities of longitudinal and transverse ultrasonic waves on concrete cores bored out of an existing concrete structure, and studied how the sonic velocity ratio between the longitudinal and transverse waves related to the compressive strength and the progress of alkali aggregate reaction. The inventor found that the sonic velocity ratio between the longitudinal and transverse waves was in a proportional relationship to the compressive strength of concrete (see Fig. 3) and was also closely correlated to the progress of alkali aggregate reaction (see Fig. 4).
It is therefore seen that, if the compressive strength of and the progress of alkali aggregate reaction in a given concrete structure are unknown, its object physical quantities can be found out in a completely non-destructive way without going into the trouble of core boring and extraction or the like by measuring on site the sonic velocities of the longitudinal and transverse waves, applying the sonic velocity ratio between the longitudinal and transverse waves thereby obtained to the diagram of the aforementioned correlation, even if the concrete structure does not permit application of ultrasonic transmission.
However, in order to measure by ultrasonic probing the sonic velocities of the transverse and longitudinal waves at specific measuring points in a concrete structure, usually it is necessary to measure each individual point with a dedicated probe for longitudinal waves and a dedicated probe for transverse waves. This means a problem that the sonic velocity ratio cannot be immediately obtained on site but takes a very long time to determine. Measurement with a dedicated probe for transverse waves is particularly troublesome because a specific contact medium should intervene between the probe and the object of testing.
An object of the present invention is to provide, in view of the problems noted above, a method and apparatus for non-destructive testing of concrete structures that permit ready on-site measurement of object physical quantities of a concrete structure, such as compressive strength and progress of alkali aggregate reaction, by measuring eachmeasuring point only once to determine the sonic velocity ratio between the longitudinal and transverse waves.
The present inventor, through his experience of ultrasonic probing of many concrete structures using a dedicated probe for longitudinal waves, noticed that transverse waves generated by mode conversion on a boundary even in concrete structures are included in waveform data of reflected waves, and this finding led to the completion of this invention.

Thus, the invention provides a method for non-destructive testing of concrete structures comprising the steps of:

(a) applying an ultrasonic pulse from a transmission probe for longitudinal waves kept in contact with the external surface of a concrete structure into the interior of the structure, and detecting with a reception probe for longitudinal waves kept in contact with the external surface of a concrete structure a reflected wave returning after being reflected by the boundary face of the structure;

(b) calculating the sonic velocity ratio between the longitudinal and transverse waves from the reaching times of bottom face echo and the reaching time of delayed echo, as, out of a plurality of peaks recognized in the waveform of the reflected waves, the peak in the position of frequency changing greatly (1st peak) represents the reaching time of bottom face echo which has undergone no mode conversion, and the subsequently obtained peak, in about 1.3 to 1.7 times when the 1st peak appeared represents the reaching time of delayed echo which has undergone a mode conversion, and

(c) figuring out, from correlations known in advance regarding the concrete structure between the sonic velocity ratio and object physical quantities, the object physical quantities corresponding to the sonic velocity ratio measured on site.

According to the invention described above, out of a plurality of peaks recognized in the waveform of the reflected waves, the first appearing peak represents the reaching time of the bottom face echo which has undergone no mode conversion, and the subsequently obtained peak represents the reaching time of the delayed echo which has undergone a mode conversion. As the sonic velocity ratio between the longitudinal and transverse waves is calculated from these two different reaching times, the sonic velocity ratio of any specific measuring point of the concrete structure can be figured out by only one measuring action. As a result, there is no need for separate measuring with a dedicated probe for longitudinal waves and another dedicated probe for transverse waves.

For this reason, object physical quantities regarding the concrete structure corresponding to the sonic velocity ratio simply measured on site as stated above can be obtained very quickly from the correlations between the sonic velocity ratio and the object physical quantities known in advance regarding concrete structures.

In the context of the invention, object physical quantities regarding the concrete structure include all the factors correlated to the sonic velocity ratio between the longitudinal and transverse waves. As will be described afterwards, since it is known from laboratory-measured data on concrete cores that the sonic velocity ratio between the longitudinal and transverse waves is proportional to the compressive strength and is also closely correlated to the progress of alkali aggregate reaction in the concrete structure, at least these two factors, namely the compressive strength and the progress of alkali aggregate reaction, are included in the object physical quantities.

Further, supposing that a concrete structure is a homogeneous isotropic body, taking a macroscopic view, as the elasticity constant (Young's modulus) can be calculated from the density of the concrete structure, the sonic velocity of longitudinal waves and the sonic velocity ratio, and the Poisson ratio can be calculated from the sonic velocity ratio, with the result that these Young's modulus and Poisson ratio are also included in the object physical quantities.

On the other hand, according to the invention, by applying a plurality of ultrasonic pulses onto a concrete structure from the transmission probe, receiving the reflected waves of the ultrasonic pulses with the reception probe, and adding and averaging on the same time axis waveforms resulting from the digitization of the received signals, the reflected waves from the target of probing can be amplified while removing scattering which obstructs the probing, and the accuracy of measurement can be further improved.

If in that process the reflected waves are detected while moving either one or both of the two probes along the external surface of the concrete structure, even if there are relatively large masses of crude aggregate or gaps behind the measuring point into which the probe comes into contact, applied ultrasonic pulses will not keep on hitting against those obstacles. For this reason, the influence of those obstacles on the reflected waves will stay on, and peaks of the output waveform will become even clearer.

Therefore, in implementing the measuring method described above, in order to minimize the influence of crude aggregate in the concrete on the reflected waves, it is preferable to set the moving range of the probes to be greater than the expected size of the crude aggregate in the concrete (about 3.0 to 5.0 cm). It is also preferable, where the transmission probe and the reception probe are different units independent of each other, to move these probes separately.

The reason is that moving the transmission probe prevents the ultrasonic pulses immediately after coming into incidence from keeping on hitting against any obstacle, moving the reception probe prevents the echoes immediately after reception from keeping on hitting against any obstacle, and the influence of any obstacle in the concrete can be made smaller than when only one of the probes is moved.

Meanwhile in the measuring method described above, there is no restriction on the way of moving the probes. For instance, they may be reciprocated in a fixed direction, vertical or horizontal, or randomly moved within a prescribed range on the surface of the concrete structure. As will become more apparent in the subsequent description of the preferred embodiment of the invention, in order to minimize the adverse influence of any obstacle in the concrete, it is more preferable to move the probes at random.

As described above, the present invention enables the sonic velocity ratio between the longitudinal and transverse waves to be determined by measuring each measuring point only once, and therefore makes possible ready on-site measurement of object physical quantities of concrete structures, such as the compressive strength and the progress of alkali aggregate reaction.

BRIEF DESCRIPTION OF THE DRAWINGS

The above-described and other objects and features of the present invention will become more apparent from the following description of a preferred embodiment thereof when taken in conjunction with the accompanying drawings, wherein:

Fig. 1 schematically shows the configuration of a non-destructive testing apparatus for implementing the method according to the invention;

Fig. 2 shows one example of output waveform obtained in actual measurement carried out by using the non-destructive testing apparatus of Fig. 1;

Fig. 3 is a graph showing the correlation between the sonic velocity ratio between longitudinal and transverse waves and compressive strength; and

Fig. 4 is a graph showing the correlation between the progress of alkali aggregate reaction and the sonic velocity ratio between longitudinal and transverse waves.

DETAILED DESCRIPTION OF THE PREFERRED EMBODIMENTS

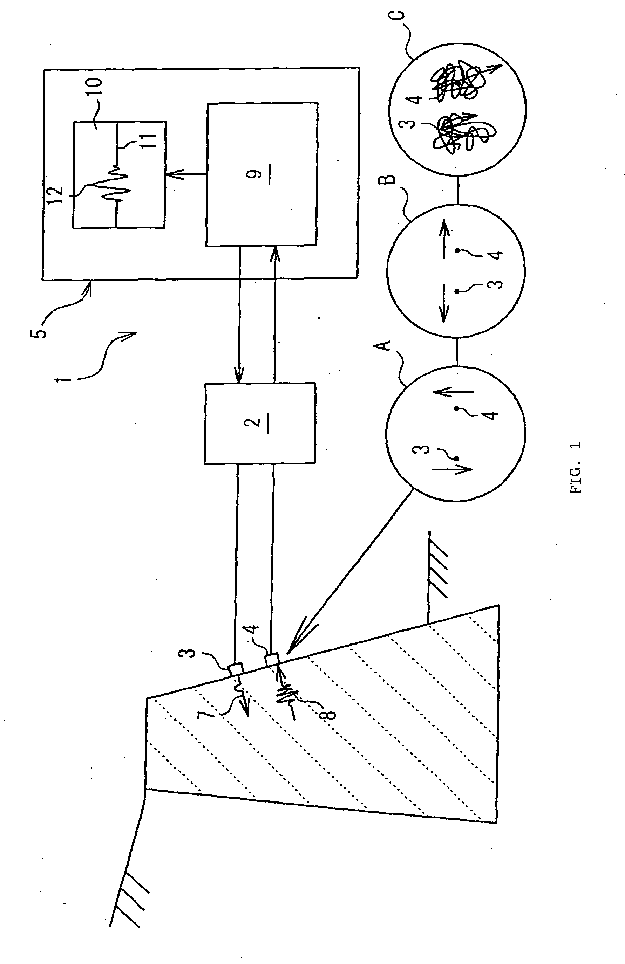

Fig. 1 schematically shows the configuration of a non-destructive testing apparatus, which is an embodiment of the present invention. As shown in this drawing, a non-destructive testing apparatus 1 comprises a non-destructive testing apparatus body 2, a dedicated transmission probe (pulsar) 3 for longitudinal waves connected to this testing apparatus body 2, a dedicated reception probe (receiver) 4 for longitudinal waves connected to the testing apparatus body 2 separately from this transmission probe 3, and a data processing apparatus 5, composed of a portable personal computer or the like, connected to the testing apparatus body 2.

Of these elements, the transmission probe 3 transmits ultrasonic pulses 7 into a concrete structure, which may be a sheathing revetment, a weir or the like. The transmission probe 3 is configured by stacking acoustic matching layers comprising epoxy resin or the like over a piezoelectric element comprising an electro-acoustic conversion element composed of piezoelectric ceramic, piezoelectric macromolecule or the like.

The reception probe 4 receives the echoes 8 of the ultrasonic pulses 7 on the same plane as the transmitting side. The reception probe 4 has basically the same structure as the transmission probe 3. Each of the probes 3 and 4 has an ultra-wide band ranging from audible region of around 10 kHz to usual defect detectors' frequency band of around 5 MHz.

The testing apparatus body 2 is provided with a transmission control circuit for controlling the timing of transmission of the ultrasonic pulses 7 by the transmission probe 3, a main amplifier for amplifying signals from the reception probe 4, an A/D converter circuit for digitizing the signals amplified by this amplifier, and an adder for adding and averaging the digitized waveforms on the same time axis.

On the other hand, the data processing unit 5 comprises an auxiliary memory device consisting of a hard disk or the like on which an OS, prescribed application programs and the like are permanently stored; an auxiliary memory device (main RAM) for temporarily storing those programs and data; a personal computer body 9 comprising a CPU and.the like for operating and controlling those programs and data; and a display unit 10 comprising a liquid crystal display or the like connected to this body 9. Applicationprograms for giving instructions on the setting of transmitting, digital filtering of data and so forth to the testing apparatus body 2 are installed in the personal computer body 9.

Therefore, the non-destructive testing apparatus 1 of this embodiment can apply a plurality of ultrasonic pulses 7 into a concrete structure 6 from the transmission probe 3, receive the echoes 8 of the ultrasonic pulses 7 with the reception probe 4, and display on the display unit 10 of the data processing unit 5 an output waveform 11 obtained by adding and averaging on the same time axis waveforms resulting from the digitization of those received signals.

A program stored in the auxiliary memory device of the personal computer body 9 contains as data the relationship between the sonic velocity ratio between longitudinal and transverse waves and the compressive strength of concrete (Fig. 3) and the relationship between the progress of the alkali aggregate reaction of concrete and the sonic velocity ratio of the same (Fig. 4).

These data were obtained from many concrete cores ($\phi$ 100 mm and H = 200 mm) bored and extracted from a structure different from the concrete structure 6 to be measured by measuring in the laboratory the ultrasonic velocities of longitudinal waves and of transverse wave by using the transmission method and determining the compressive strength of and the progress of the alkali aggregate reaction in each core. Regarding the compressive strength, as three to five cores could be collected in each extracting position, the average of the individual compressive strengths was used as the compressive strength in the pertinent extracting position.

On the other hand, since no generally applicable standards for quantifying the progress of the alkali aggregate reaction have been established as yet, the states of the aggregate on the transverse plane along which each core was sliced were classified into the following stages, and points were assigned by weighting 10 positions.

· Reaction stage 1: 2 points per position (white dots are found on the aggregate.)
· Reaction stage 2: 4 points per position (the aggregate is surrounded by a white or black ring.)
· Reaction stage 3: 8 points per position (cracks are found in the aggregate.)
· Reaction stage 4: 16 points per position (cracks in the aggregate extend to the mortar part.)

Therefore, if all the 10 positions on a given sectional plane are at reaction stage 1, the weight will be 20 (= 10 x 2) points, or if all are at reaction stage 4, it will be 160 (= 10 x 16) points.

As shown in Fig. 3, the sonic velocity ratio between longitudinal and transverse waves is in a proportional relationship to the compressive strength of concrete and, as shown in Fig. 4, there is a close correlation between the progress of the alkali aggregate reaction and the compressive strength; the faster the former, the lower the latter.

Further, with the concrete structure 6 being supposed to be an isotropic homogeneous elastic body, the elasticity constant (Young's modulus) can be calculated by Equation (1) below, and the Poisson ratio can be calculated by Equation (2) below. In these equations, E represents the elasticity constant; R, the sonic velocity ratio; p, the density; Vt, the sonic velocity of transverse waves; and u, the Poisson ratio.

$$E = \rho Vt^2 (3 - (1/(R^2 - 1))) \quad \ldots \ (1)$$

$$\upsilon = (1/2)(1 - (1/R^2 - 1)) \quad \ldots \ (2)$$

Then, in this embodiment of the invention, the program stored in the auxiliary memory device of the personal computer body 9 also contains as data Equations (1) and (2) above for figuring out the elasticity constant and the Poisson ratio.

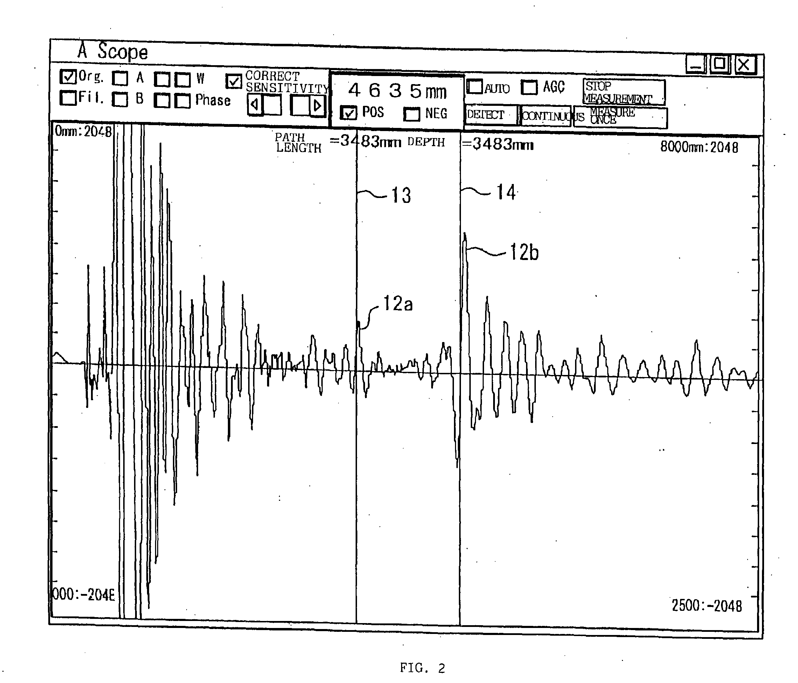

Incidentally, during repeated attempts to measure the thickness of the concrete structure 6 by ultrasonic probing, the present inventor noticed that, even when only the dedicated probes 3 and 4 for the longitudinal waves were used, a second peak 12b significantly appeared after the first peak 12a as shown in Fig. 2, and suspected that this second peak 12b could be attributed to the influence of transverse waves generated by a mode conversion on the boundary face.

Then, as shown in Fig. 2, a first cursor 13 was positioned in the position of the horizontal axis (reaching time) in the case in which waves are longitudinal (4000 m/s in supposed sonic velocity) both ways on the screen of waveform data, and a second cursor 14 was positioned in the position of the horizontal axis in the case in which longitudinal waves were dominant forward and transverse waves (2350 m/s in supposed sonic velocity) were dominant on the way back. The first cursor 13 was found substantially coinciding with the peak 12a and the second cursor 14 was found well coinciding with the second peak 12b.

Therefore it was found out that, even when the concrete structure 6 was ultrasonically probed with only the dedicated probes 3 and 4 for longitudinal waves, the echoes of transverse waves generated by the mode conversion on the boundary face were picked up by the dedicated reception probes for the longitudinal wave and were contained in the waveform data of the reflected waves.

For this reason in this embodiment, the sonic velocity ratio between longitudinal and transverse waves is calculated from the reaching time T1 of the first obtained bottom face echo which has undergone no mode conversion, and the reaching time T2 of the subsequently obtained delayed echo which has undergone a mode conversion, out of a plurality of peaks 12a and 12b recognized in the waveform of the reflected waves.

Thus, since it can be assumed that the first peak 12a represents the bottom face echo which has undergone no mode conversion and the second peak 12b represents the echo of the transverse waves generated by mode conversion on the boundary face, the sonic velocity ratio R (= V1/V2) of longitudinal wave to transverse wave can be expressed in Equation (3) below, wherein the reaching time of the former is represented by T1 and that of the latter is represented by T2.

$$R= (2 \times T2 - T1)/T1 \quad \ldots (3)$$

And in this embodiment, Equation (3) above is contained as data in the program stored in the auxiliary memory device of the personal computer body 9, and the sonic velocity ratio R can be calculated by substituting the reaching times T1 and T2, measured on site, into this Equation (3).

Next will be described the method by which object physical quantities including the compressive strength of and the progress of the alkali aggregate reaction in the concrete structure 6 are measured by using the non-destructive testing apparatus 1.

First, an operator manually brings the transmission probe 3 and the reception probe 4 into contact with the surface of the concrete structure 6 at the point where such object physical quantities are desired to be measured. Then, ultrasonic pulses 7 are generated at a prescribed output voltage and applied onto the concrete structure 6, and the step of measuring an echo 8 caused by each of ultrasonic pulses 7 is repeated many times (100 to 1000 times, for instance).

While these echoes 8 are being received, either one or both of the transmission probe 3 and the reception probe 4 are moved along the surface of the concrete structure 6, for instance as indicated in circles A to C in Fig. 1.

Incidentally, the probes 3 and 4 may be reciprocated vertically as indicated in circle A in Fig. 1. Or they may, be reciprocated horizontally as indicated in circle B. Or the probes may be randomly moved within a prescribed range on the surface of the concrete structure as indicated in circle C.

Further, in order to minimize the influence of crude aggregate in the concrete on the reflected waves, it is preferable to set the moving range of the probes 3 and 4 to be greater than the expected size of the crude aggregate (about 3.0 to 5.0 cm), and it is also preferable to apply liquid lubricant to the concrete structure 6 at the measurement range in advance to prevent the otherwise possible influence of scattered waves due to faulty contact. The preferable moving speed of the probes 3 and 4 is approximately 5 to 12 cm/sec.

The echoes 8 obtained by the repeated measuring steps described above are digitized by the testing apparatus body 2, and the output waveform 11 obtained by adding and averaging the resultant digital waveforms on the same time axis is displayed on the display unit 10 of the data processing unit 5.

This arithmetic averaging cancels any noise contained in each of the echoes 8 and thereby provides an output waveform 11 of a high S/N ratio. Also, if the amplitude is either too large or too small, an appropriate amplitude can be obtained by increasing or decreasing the number of times of addition or the output voltage.

By the first peak 12a appearing in the output waveform 11 on the display unit 10, the position of the boundary face between the concrete structure 6 and a natural ground 16 behind it is measured, the thickness of the structure 6 is determined, and the reaching time T1 of the first peak 12a and the reaching time T2 of the second peak 12b are detected.

Then, by substituting these reaching times T1 and T2 into Equation (3) above, the sonic velocity ratio R of longitudinal wave to transverse wave can be figured out. Further, the compressive strength corresponding to this sonic velocity ratio R is figured out from the correlation diagram shown in Fig. 3 and the progress of alkali aggregate reaction corresponding to the sonic velocity ratio R is figured out from the correlation diagram shown in Fig. 4.

To add, the elasticity constant (Young's modulus) and the Poisson ratio, if needed, can be calculated by substituting the sonic velocity ratio R and the transverse wave velocity Vt into Equations (1) and (2).

As hitherto described, according to the non-destructive testing method embodying the invention in this way, the sonic velocity ratio R of longitudinal wave to transverse wave is calculated from the reaching time T1 of the first obtained bottom face echo which has undergone no mode conversion (the peak 12a), and the reaching time T2 of the subsequently obtained delayed echo which has undergone a mode conversion (the peak 12b), out of a plurality of peaks 12a and 12b recognized in the waveform of the reflected waves. Therefore, the sonic velocity ratio R of any specific measuring point of the concrete structure 6 can be figured out by only one measuring action. As a result, obj ect physical quantities regarding the concrete structure 6 corresponding to the sonic velocity ratio R can be obtained very quickly from the correlations between the sonic velocity ratio R and the object physical quantities known in advance (Fig. 3 and Fig. 4 and Equations (1) and (2)).

The present invention is not confined to its embodiment described above, but, for instance, a probe for longitudinal waves applicable to both transmission and reception by itself can be used as well.

**Claims**

**1.** A non-destructive testing method for concrete structures, comprising the steps of:

> (a) applying an ultrasonic pulse from a transmission probe for longitudinal waves kept in contact with the external surface of a concrete structure into the interior of the structure, and detecting with a reception probe for longitudinal waves kept in contact with the external surface of a concrete structure a reflected wave returning after being reflected by the boundary face of the structure;

(b) calculating the sonic velocity ratio between longitudinal and transverse waves from the reaching times of a bottom face echo which has undergone no mode conversion, determined by the first appearing peak, and the reaching time of delayed echo which has undergone a mode conversion, determined by the subsequently obtained peak, out of a plurality of peaks recognized in the waveform of the reflected waves; and

(c) figuring out, from correlations known in advance regarding said concrete structure between the sonic velocity ratio and object physical quantities, said object physical quantities corresponding to say sonic velocity ratio measured on site.

2. The non-destructive testing method for concrete structures according to claim 1, wherein said object physical quantities are compressive strength, progress of alkali aggregate reaction and elasticity constant or Poisson ratio of said concrete structure.

3. The non-destructive testing method for concrete structures according to claim 1, wherein said step (a) includes applying a plurality of ultrasonic pulses from said transmission probe onto said concrete structure at a prescribed timing; receiving each of the reflected waves of the ultrasonic pulses with said reception probe, and adding and averaging on the same time axis a waveform resulting from the digitization of those received signals.

4. The non-destructive testing method for concrete structures according to claim 1, wherein said reflected waves are detected while moving either one or both of said probes along the external surface of said concrete structure.

5. A non-destructive testing apparatus for concrete structures, comprising:

a transmission probe for longitudinal waves for applying an ultrasonic pulse from the external surface of a concrete structure into the interior thereof;

a reception probe for longitudinal waves for detecting a reflected wave returning after being reflected by the boundary face of said concrete structure;

arithmetic means for calculating the sonic velocity ratio between longitudinal and transverse waves from the reaching times of a bottom face echo which has undergone no mode conversion, determined by the first appearing peak, and the reaching time of delayed echo which has undergone a mode conversion, determined by the subsequently obtained peak, out of a plurality of peaks recognized in the waveform of the reflected waves;

memory means for storing correlations between a sonic velocity ratio obtained in advance regarding said concrete structures and object physical quantities which are to be examined; and

means of figuring out, from the correlations stored in said memory means, said object physical quantities corresponding to say sonic velocity ratio measured on site.

6. The non-destructive testing apparatus for concrete structures according to claim 5, wherein said obj ect physical quantities are compressive strength, progress of alkali aggregate reaction and elasticity constant or Poisson ratio of the concrete structure.

7. The non-destructive testing apparatus for concrete structures according to claim 5, wherein saidmeans of figuring out the object physical quantities has functions to applying a plurality of ultrasonic pulses from said transmission probe into said concrete structure at a prescribed timing; to receive each of the echoes of the ultrasonic pulses with said reception probe, and to add and average on the same time axis a waveform resulting from the digitization of those received signals.

8. A computer program for controlling a non-destructive testing apparatus for concrete structures which comprises a testing apparatus body, a transmission probe for longitudinal waves and a reception probe for longitudinal waves connected to this testing apparatus body and a programmable data processing unit connected to say testing apparatus body,

wherein said program causes a computer to execute steps referred to in any of claims 1 through 4.

9. A computer-readable storage medium storing the program referred to in claim 8.

FIG. 1

EP 1 780 539 A1

FIG. 2

FIG. 3

FIG. 4

**European Patent Office**

**EUROPEAN SEARCH REPORT**

Application Number

EP 05 25 6645

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 6 311 565 B1 (HINZ WILLIAM R ET AL) 6 November 2001 (2001-11-06) | 1,3-5, 7-9 | G01N29/04 G01N29/07 |
| A | * abstract; claims 1,5; figures 1-3,7,8 * * column 2, line 17 - column 41 * * column 4, line 38 - column 6, line 58 * ----- | 2,6 | G01N29/38 G01N29/44 G01N33/38 |
| Y | DATABASE WPI Section EI, Week 198144 Derwent Publications Ltd., London, GB; Class S03,page 003, AN 1981-L3596D XP002374777 LAPENIS A T I: "Setting concrete strength meter" & SU 794 476 A (PT TREST "ORGTEKHSTROJ") 7 January 1981 (1981-01-07) * abstract * ----- | 1,3-5, 7-9 | |
| D,A | PATENT ABSTRACTS OF JAPAN vol. 2003, no. 12, 5 December 2003 (2003-12-05) & JP 2003 302382 A (FUJIMITSU KOMUTEN:KK; OGAWA FUJIO), 24 October 2003 (2003-10-24) * abstract * ----- | 1,5 | |
| A | STEBER G R ET AL: "Detection and processing of impact echoes in concrete" SIGNAL PROCESSING AND SYSTEM CONTROL, FACTORY AUTOMATION. PACIFIC GROVE, NOV. 27 - 30, 1990, PROCEEDINGS OF THE ANNUAL CONFERENCE OF THE INDUSTRIAL ELECTRONICS SOCIETY. (IECON), NEW YORK, IEEE, US, vol. VOL. 1 CONF. 16, 27 November 1990 (1990-11-27), pages 340-345, XP010038158 ISBN: 0-87942-600-4 * the whole document * ----- | 1,5 | |

TECHNICAL FIELDS SEARCHED (IPC)

G01N

The present search report has been drawn up for all claims

| Place of search | Date of completion of the search | Examiner |
|---|---|---|
| Munich | 29 March 2006 | Uttenthaler, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 05 25 6645

This annex lists the patent family members relating to the patent documents cited in the above-mentioned European search report.
The members are as contained in the European Patent Office EDP file on
The European Patent Office is in no way liable for these particulars which are merely given for the purpose of information.

29-03-2006

| Patent document cited in search report | | Publication date | Patent family member(s) | | Publication date |
|---|---|---|---|---|---|
| US 6311565 | B1 | 06-11-2001 | AU<br>WO | 2404900 A<br>0041517 A2 | 01-08-2000<br>20-07-2000 |
| SU 794476 | A | 07-01-1981 | NONE | | |
| JP 2003302382 | A | 24-10-2003 | NONE | | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003302382 A